# EUROPEAN PATENT SPECIFICATION

(11) **EP 2 518 075 B1**
(45) Date of publication and mention of the grant of the patent: **08.07.2015**
(21) Application number: 10838498.3
(22) Date of filing: 03.08.2010
(51) Int. Cl.: C07D 333/10, C07D 333/18, C07D 333/76, C07D 495/04, C07C 15/14, C07C 15/30, H01B 1/12, H01L 51/00

(54) **INDUCED LAYER MATERIAL FOR PREPARING NON-PLANAR METAL PHTHALOCYANINE WEAK EPITAXY GROWTH THIN FILM**
INDUZIERTES SCHICHTMATERIAL ZUR HERSTELLUNG EINES NICHT PLANAREN PHTHALOCYANIN-DÜNNFILMS MIT SCHWACHEM EPITAXIE-WACHSTUM
MATÉRIAU À COUCHE INDUITE DESTINÉ À PRÉPARER UN FILM MINCE À CROISSANCE ÉPITAXIALE FAIBLE DE PHTALOCYANINE MÉTALLIQUE NON PLANAIRE

(30) Priority: 22.12.2009 CN 200910200459
(43) Date of publication of application: 31.10.2012
(73) Proprietor: Changchun Flexible Display Technology Co., Ltd., Changchun Jilin 130012 (CN)
(72) Inventor: YAN, Donghang, Shanghai 201203 (CN); GENG, Yanhou, Shanghai 201203 (CN); TIAN, Hongkun, Shanghai 201203 (CN); PAN, Feng, Shanghai 201203 (CN); HUANG, Lizhen, Shanghai 201203 (CN)
(74) Representative: Regimbeau
(86) International application number: PCT/CN2010/001174
(87) International publication number: WO 2011/075932

(56) References cited:
- CN-A- 101 562 230
- YANAGI, HISAO ET AL: "Comparative carrier transport characteristics in organic field-effect transistors with vapor-deposited thin films and epitaxially grown crystals of biphenyl-capped thiophene oligomers", ADVANCED FUNCTIONAL MATERIALS , 13(10), 767-773 CODEN: AFMDC6; ISSN: 1616-301X, 2003, XP001174827,
- NOH YONGYOUNG ET AL.: 'Organic Field Effect Transistors Based on Biphenyl, Fluorene nd-Capped Fused Bithiophene Oligomers' CHEM. MATER. vol. 17, no. 15, 2005, pages 3861 - 3870
- WANG HAIBO ET AL.: 'High Mobility Vanadyl-Phthalocyanine Polycrystalline Films for Organic Field-Effect Transistors' APPLIED PHYSICS LETTERS vol. 90, 2007, pages 253510 - 1-253510-3, XP012095425

## Description

### TECHNICAL FIELD

The present invention relates to inducing layer materials for the preparation of weak epitaxial films of non-planar metal phthalocyanine.

### BACKGROUND TECHNOLOGY

In recent years , the development of organic semiconductor materials with high carrier mobility is very active, and the materials show some broad application prospects in information display,integrated circuit,photovoltaic cells and sensors, etc. Wang Haibo,etc. (Adv. Mater., 2007, 19, 2168-2171) first reported a method to prepare polycrystalline thin films of discoid organic semiconductor on the amorphous substrate-Weak Epitaxy Growth , and the field-effect carrier mobility of the polycrystalline thin films thereof reaches the level of corresponding single crystal. The principle of Weak Epitaxy Growth is to prepare a layer of organic ultra-thin film with highly ordered arrangement as inducing layer on amorphous substrate firstly, and the discoid organic semiconductor molecules nucleated orientationally on the inducing layer, and form a high quality polycrystalline thin film with large crystal domain size, highly oriented domain and good coalescene of polycrystal domain. Wang Tong, etc. (J. Phy. Chem. B, 2008, 112, 6786-6792.) reported the Weak Epitaxy Growth behavior of planar metal phthalocyanine on the para-hexaphenyl inducing layer. The planar metal phthalocyanine shows two sets of orientation with incommensurate epitaxy and one set of orientation with commensurate epitaxy on single molecular inducing layer, and shows one set of orientation with commensurate epitaxy on bimolecular inducing layer. These growth habits have a close relationship with the lattice mismatch between the epitaxial crystal and the inducing layer. Wang Haibo, etc. (Appl. Phys. Lett., 2007, 90, 253510.) reported the Weak Epitaxy Growth of vanadyl phthalocyanine which belonging to non-planar metal phthalocyanine on para-hexaphenyl inducing layer, wherein incommensurate epitaxy between them was found. Noh Yongyoung et al. ("Organic Field Effect Transistors Based on Biphenyl, fluorine nf-Capped Fused Bithiophene Oigomers", Chemistry of materials, vol. 17, no. 15, 2005, pages 3861-3870), discloses biphenyl bithiphene (BPTT), its preparation method. Yanagi, Hisao et al. ("comparative carrier transport characteristics in organic field-effect transistors with vapor-deposited thin films and epithaxially grown crystals of biphenyl-capped thiophene oligomers", Advanced Functional Materials, vol. 13(10), 767-773) discloses diphenyl-terthiophene (BP3T), diphenyl tetrathiophene (P4T), and the comparative carrier transport characteristics in organic field- effect transistors with vapor-deposited thin films and epithaxially grown crystals of biphenyl-capped thiophene oligomers. There are significant differences in parameter and type of the unit cell between non-planar metal phthalocyanine and planar metal phthalocyanine, and their Weak Epitaxy Growth habits on para-hexaphenyl inducing layer are significantly changed, so the lattice matching between them is difficult. In order to obtain high-quality polycrystalline thin films of organic semiconductors of non-planar metal phthalocyanine, it is needed to develop a new type of inducing layer materials whose lattice can be matched with that of the non-planar metal phthalocyanine.

### CONTENT OF THE INVENTION:

In order to overcome the deficiencies of the prior art , the purpose of the present invention is to provide a series of new type of inducing layer materials which are suitable for the Weak Epitaxy Growth of non-planar metal-phthalocyanine.

The principle of the present invention is to replace benzene rings of para-hexaphenyl by conjugated aromatic groups (polycyclic aromatic compounds or oligomers with 2 to 4 aromatic rings containing benzene ring and thiophene ring) and/or to replace the hydrogen atoms of benzene rings at the two ends of *para-*hexaphenyl by fluorine atoms , thus the regulation of molecular interaction is finally realized by changing the size or the linearity degree of the conjugated aromatic groups, as well as by changing the polarity of the benzene ring at the two ends, consequently, the cell parameters of (001) crystal face of the new materials are different from that of *para*-hexaphenyl and can match the cell parameters of non-planar metal phthalocyanine. The molecular structure of the new inducing layer materials is shown as the following general formula:

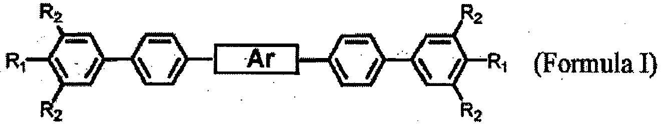

Ar in general formula I is a conjugated aromatic group, including the following structures:

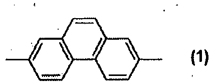

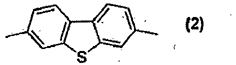

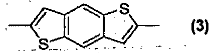

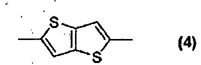

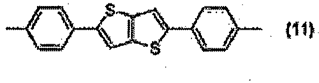

R1 and R2 in general formula I are hydrogen atoms (H) or fluorine atoms (F). According to the differences of the chemical structures of R1 and R2, the molecular structures of the new inducing layer materials conformed to general formula I can be divided into the following three types :
(1) R1= R2=H, the specific chemical structures, names and initials of the compounds are listed in Table 1.

**Table 1**

| |
|---|
| 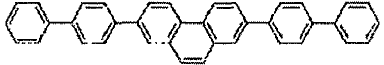 |
| 2,7 - di (4 - biphenyl) -phenanthrene (BPPh) |
| 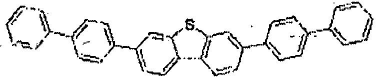 |
| 2,7 - di (4 - biphenyl) - dibenzothiophen (BPBTB) |
| 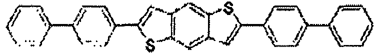 |
| 2,6 - di (4 - biphenyl) - benzo [1,2-*b* ;4,5-*b'*] bithiophene (BPTBT) |
| 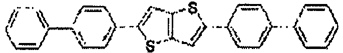 |
| 2,5 - di (4 - biphenyl) - thieno[3,2-b]thiophene (BPTT) |
| 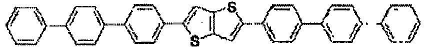 |
| 2,5 - di(4-1,1':4',1"-terphenyl)-thieno[3,2-b]thiophene (3PTT) |

(2) Ri=F, R2=H, specific chemical structures, names and initials of the compounds are listed in Table 2.

**Table 2**

| |
|---|
| 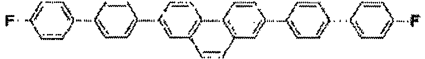 |
| 2,'7 - di (4 - 4' - fluorobiphenyl)- phenanthrene (F2-BPPh) |
| 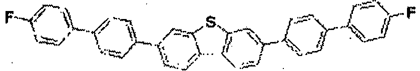 |
| 2,7 - di ( 4 - 4' - fluorobiphenyl) - dibenzothiophen (F2-BPBTB) |
| 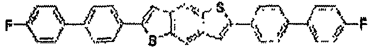 |
| 2,6 - di (4 - 4' - fluorobiphenyl) - benzo[1,2,*b*:4,5-*b'*] bithiophene (F2-BPTBT) |
| 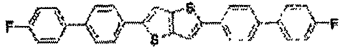 |
| 2,5 - di (4 - 4' - fluorobiphenyl) - thieno[3,2-b]thiophene (F2-BPTT) |
| 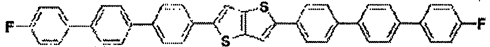 |
| 2,5 - di (4 - 4" - fluoro - 1,1':4',1" - terphenyl)) - thieno[3,2-b]thiophone (F2-3PTT) |

(3)R₁=H, R₂=F, specific chemical structures, names and initials of the compounds are listed in Table 3

**Table 3**

| |
|---|
| 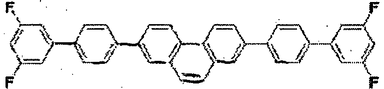 |
| 2,7 - di (4 - 3',5' - bifluorobiphenyl) - phenanthrene (F4-BPPh) |
| 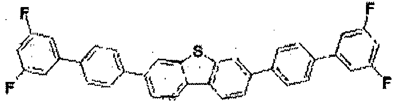 |
| 2,'7 - di(4 - 3',5' - bifluorobiphenyl) - dibenzothiophen(F4-BPBTB) |
| 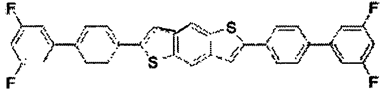 |
| 2,6 - di (4 - 3' ,5' - bifluorobiphenyl) - benzo[1,2-*b*:4,5-*b'*] bithiophene (F4-BPTBT) |
| 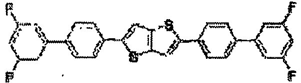 |
| 2,5 - di (4 - 3',5' - bifluorobiphenyl) - thieno[3,2-b]thiophene (F4-BPTT) |
| 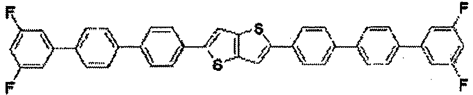 |
| 2,5 - di (4 - 3",5" - bifluoro - 1,1':4',1" - terphenyl) - thieno[3,2-b]thiophene (F4-3PTT) |

BPTT listed in Table 1 are prepared in accordance with the technical solutions in the prior art, as follows: BPTT(Chem. Mater., 2005, 17, 3861-3870.). BPPh , BPBTB and BPTBT can be produced by the Suzuki coupling reaction, and the reaction intermediates involved are industrial products or prepared in accordance with the technical solutions in the prior art , as follows: 4 - biphenyl boric acid is industrial product , 2,7 - dibromophenanthrene (Chem. Commun., 2006, 3498-3500 .), 2,7 - dibromo dibenzothiophen (J. Mater. Chem., 1999, 9, 2095-2101 .), 2,6 - diiodo - benzo[1,2-b:4,5-*b'*] bithiophene(J. Org. Chem., 1994, 59, 3077-3081.). 3PTT can be produced by Stille coupling reaction, and the reaction intermediates involved are industrial products or prepared in accordance with the technical solutions in the prior art, as follows:4 - bromo - 1,1':4',1'' - terphenyl (J. Phys. Chem. B, 2001, 105, 8845-8860 .), and 2,5-di (tributylstannyl) - thieno[3,2-b]thiophene (J. Chem. Soc., Perkin. Trans. 1, 1997, 15, 3465-3470.).

The preparation of the compounds listed in Table 2 involves a key intermediate- 4 - bromo - 4' - fluorobiphenyl , which prepared in accordance with technical solutions in the prior art (J. Chem. Soc. , 1937 , 1359). As shown in the following drawing , through the chemical reaction between 4 - bromo - 4' - fluorination biphenyl and magnesium metal, the corresponding grignard reagent can be prepared, then the grignard reagent reacts with trimethyl borate, the product is finally acidified with dilute hydrochloric acid and 4' - fluorination biphenyl - 4 - boric acid is obtained.

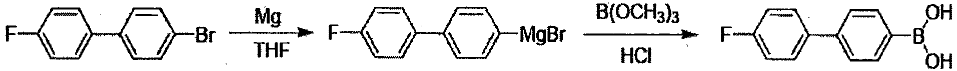

The preparation method of F2-BPPh, F2-BPBTB, F2-BPTBT is the same as that of BPPh , BPBTB , BPTBT respectively , it is only needed to replace 4 - biphenyl boric acid by 4' - fluorobiphenyl - 4 - boric acid. F2-BPTT , can be produced through Stille coupling reaction between 4 - bromo - 4' - fluoriobiphenyl and 2,5-di (tributylstannyl)- thieno[3,2-b]thiophene. The preparation methods of F2-3PTT is as follows: 4 - bromo - 4" - fluoro - 1,1':4',1"- terphenyl can be produced by Suzuki coupling reaction between 4'- fluorobiphenyl - 4 - boric acid and 1-bromo-4-iodobenzene (industrial product) (as the following drawing), the product reacts through Stille coupling reaction with 2,5-di (tributylstannyl)-thieno[3,2-b]thiophene respectively, thus F2-3PTT are correspondingly obtained 5'.

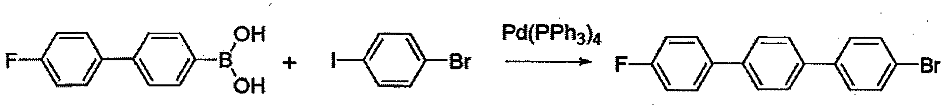

The preparations of the compounds listed in Table 3involve a key intermediate 4 - bromo - 3',5' - difluorobiphenyl , which is prepared in accordance with the technical solution in the prior art(J. Organomet. Chem. ,2000 ,598, 127-135.)as shown in the following drawing: the corresponding grignard reagent can be prepared through the chemical reaction between 3,5 - difluoro bromobenzene(industrial product)and magnesium metal, then the grignard reagent reacts with trimethyl borate, the product is finally acidified with dilute hydrochloric acid to prepare 3,5 - difluorobenzene boric acid , and 4 - bromo - 3',5' - difluorobiphenyl can be produced by Suzuki coupling reaction between 3,5 - difluorobenzene boric acid and 1-bromo-4-iodobenzene(industrial product).

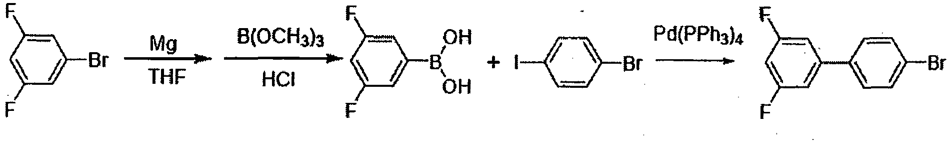

According to the preparation method of 4' - fluorobiphenyl - 4 - boric acid provided above , 3',5' - difluorobiphenyl - 4 - boric acid can be produced by replacing 4 - bromo - 4' - fluoro biphenyl with 4 - bromo - 3',5' - difluoro biphenyl.

In view of the structural differences between the compounds listed in Table 2 and Table 3 being 4' - fluorophenyl and 3',5' - difluorophenyl , on the premise that 4 - bromo - 3',5' - difluorobiphenyl and 3',5' - difluorobiphenyl - 4 - boric acid have been obtained , all the compounds listed in Table 3 are easy to be prepared by using the synthetic method of the compounds listed in Table 2.

The final pure products of all the above-mentioned inducing layer materials are produced by vacuum sublimation.

### DESCRIPTION OF FIGURES

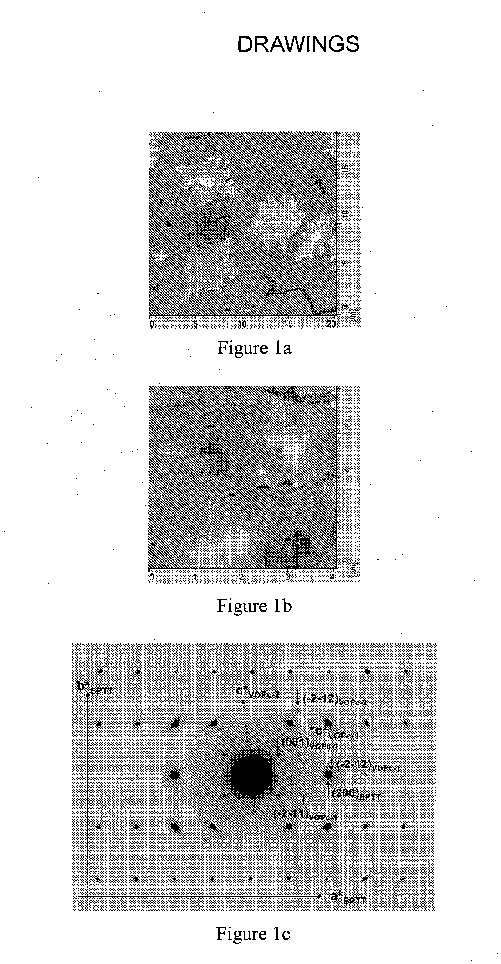

Figure 1a is an atomic force microscope image of BPTT inducing layer film , Figure 1b is an atomic force microscope image of the thin film of VOPc grew on said BPTT inducing layer film, Figure 1c is a selected area electron diffraction (SAED) pattern of the thin film of VOPc grew on said BPTT inducing layer film. Wherein the crystal face diffraction point of VOPc(-2-12) is coincident with that of BPTT(200), and there is a commensurate epitaxy relation between VOPc and BPTT in the first orientation and an incommensurate epitaxy relation in the second orientation.

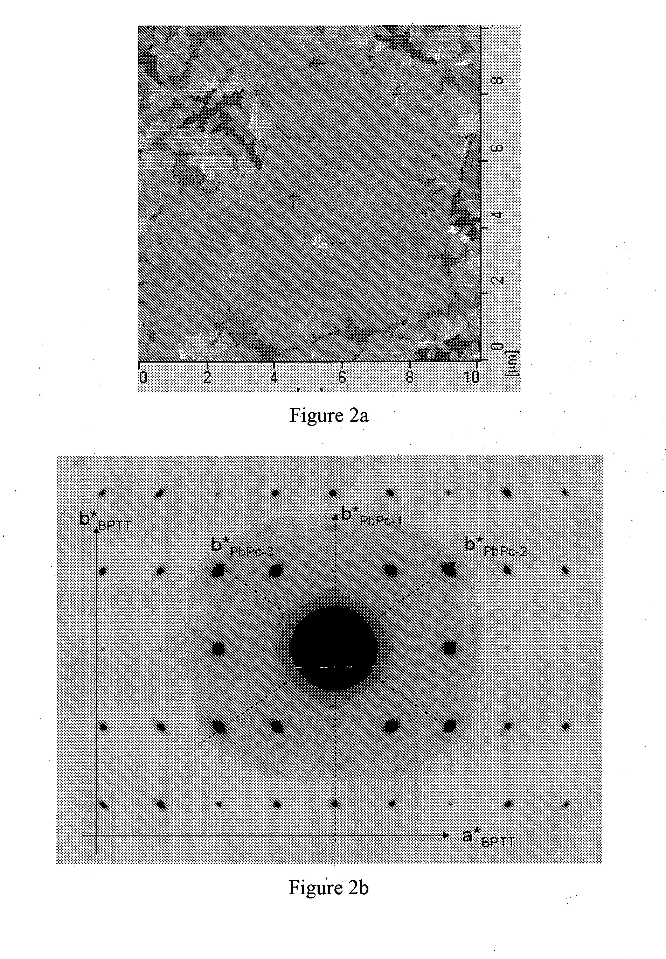

Figure 2a is an atomic force microscope image of the thin film of PbPc grew on said BPTT inducing layer film, Figure 2b is a SAED pattern of the thin film of PbPc grew on said BPTT inducing layer film. Wherein PbPc shows a commensurate epitaxy relation with BPTT in the first orientation , and shows an incommensurate epitaxy relation in the second or the third orientation.

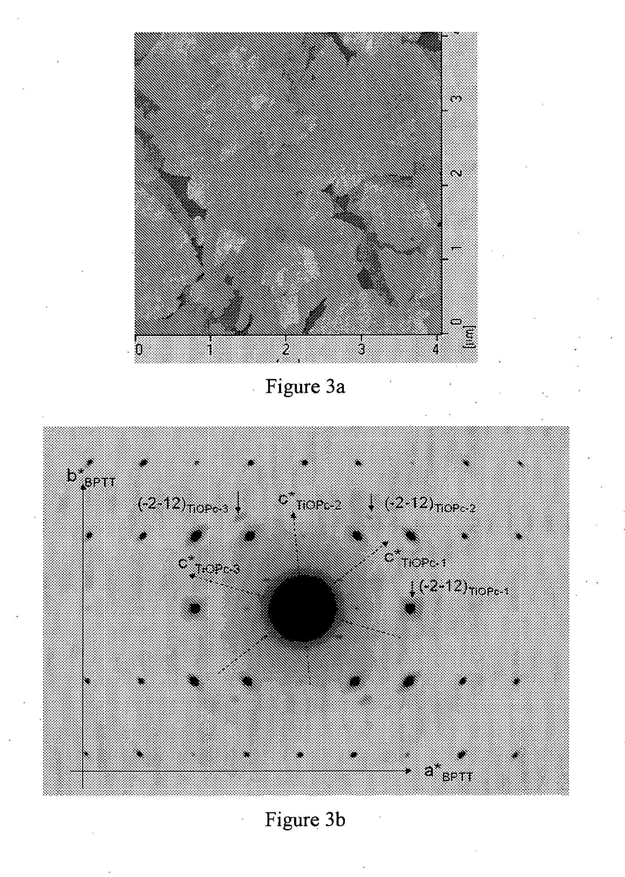

Figure 3a is an atomic force microscope image of the thin film of TiOPc grew on said BPTT inducing layer film, Figure 3b is a SAED pattern of the thin film of TiOPc grew on said BPTT inducing layer film. Wherein TiOPc shows a commensurate epitaxy relation with BPTT in the first orientation , and shows an incommensurate epitaxy relation in the second or the third orientation.

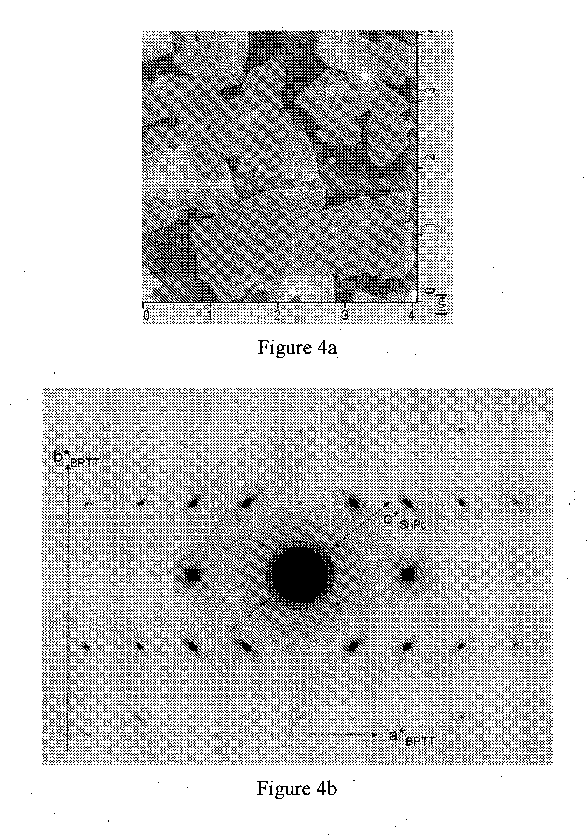

Figure 4a is an atomic force microscope image of the thin film of SnPc grew on said BPTT inducing layer film, Figure 4b is a SAED pattern of the thin film of SnPc grew on said BPTT inducing layer film, in which SnPc shows a commensurate epitaxy relation with BPTT.

### SPECIFIC WAY OF IMPLEMENTATION

The present invention is further described by combining with the example below.

### EXAMPLE:

The KANGNING 7059 glass substrate used in the experiment is commercial product , which is used after cleaned. The non-planar metal phthalocyanine used in the experiment is commercial product , which is used after purified by sublimation. The inducing layer material used in the experiment is used after purified by sublimation

First of all, silicon nitride whose thickness is about 300 nanometer grows by chemical vapor deposition method on KANGNING 7059 glass substrate. Then an inducing layer thin film whose thickness is 1 to 3 molecular layers deposited on the surface of the silicon nitride by molecular vapor deposition method, vacuum being 10⁻⁴ pa, substrate temperature being approximately 230°C.

Finally , a layer of non-planar metal phthalocyanine is deposited continuously on the inducing layer, vacuum and substrate temperature are the same as that of the inducing layer preparation.

Figure 1 a is an atomic force microscope image of 2,5 - di (4-biphenyl) - thieno[3,2-b]thiophene (BPTT) film with a thickness of one molecular layer, the film shows a smooth surface and is excellent for epitaxial growth of organic semiconductor layer.

Figure 1b is an atomic force microscope image of vanadyl phthalocyanine (VOPc) thin film with a thickness of 20 nanometers formed on the surface of said film in Figure 1a, the surface step of the VOPc molecular layer is distinct, and the VOPc film is a film with typical layer growth mode. Figure 1c is a selected area electron diffraction (SAED) pattern of the thin film of VOPc in Figure 1b, VOPc shows two orientations on BPTT. In the first orientation the crystal face diffraction point of VOPc(-2-12) is coincident with that of BPTT(200), which reveal a commensurate epitaxy relation between VOPc and BPTT. While in the second orientation, it is an incommensurate epitaxy relation between the VOPc and BPTT.

Figure 2a is an atomic force microscope image of lead phthalocyanine(PbPc) thin film with a thickness of 20 nanometers formed on the surface of said film in Figure 1a, the surface step of the PbPc molecular layer is distinct, and the PbPc film is a film of typical layer growth mode. Figure 2b is a SAED pattern of the thin film of PbPc in Figure 2a, PbPc shows three orientations on BPTT.

Wherein, there is a commensurate epitaxy relation between PbPc and BPTT in the first orientation and an incommensurate epitaxy relation in the second or the third orientation.

Figure 3a is an atomic force microscope image of titanyl phthalocyanine(TiOPc) thin film with a thickness of 20 nanometers formed on the surface of said film in Figure 1a, the surface step of the TiOPc molecular layer is distinct, and the TiOPc film is a film of typical layer growth mode. Figure 3b is a SAED pattern of the thin film of TiOPc in Figure 3a, TiOPc shows three orientations on BPTT. Wherein, there is a commensurate epitaxy relation between TiOPc and BPTT in the first orientation and an incommensurate epitaxy relation in the second or the third orientation.

Figure 4a is an atomic force microscope image of tin phthalocyanine(SnPc)thin film with a thickness of 20 nanometers formed on the surface of said film in Figure 1a, the surface step of the SnPc molecular layer is distinct, and the SnPc film is a film of typical layer growth mode. Figure 4b is a SAED pattern of the thin film of SnPc in Figure 4a, and a commensurate epitaxy relation is shown between SnPc and BPTT.

The epitaxial relationships between non-planar metal phthalocyanine and any one of the following inducing layer molecules are listed in Table 5: 2,7 - di (4 - biphenyl)-phenanthrene (BPPh), 2,7 - di (4 - biphenyl) - dibenzothiophen (BPBTB), 2,6 - di (4 - biphenyl) - benzo [1,2-b :4,5-*b'*] bithiophene(BPTBT), 2,5 - di (4 - biphenyl) - thieno[3,2-b]thiophene (BPTT), 2,5 - di (4 - 1,1':4',1" - terphenyl) - thieno[3,2-b]thiophene (3PTT), 2,7 - di (4 - 4' - fluorobiphenyl) - phenanthrene (F2-BPPh), 2,7 - di (4 - 4' fluorobiphenyl) - dibenzothiophen (F2-BPBTB), 2,6 - di (4 - 4' - fluorobiphenyl) - benzo[1,2-b:4,5-*b'*] bithiophene (F2-BPTBT), 2,5 - di (4 - 4' - fluorobiphenyl)-thieno[3,2-b]thiophene(F2-BPTT) ,5,5' - di(4 - 4' - fluorobiphenyl) - 2,2' - bithiophene (F2-BP2T), 5,5" - di (4 - 4' - fluorobiphehyl) - 2,2':5',2" - terthiophene (F2-BP3T), 2,5 - di (4 - 4" - fluoro - 1,1':4',1"-terphenyl)) - thieno[3,2-b]thiophene (F2-3PTT), 2,7 - di (4-3',5' - bifluorobiphenyl) - phenanthrene (F4-BPPh), 2,7 - di (4 - 3',5' - bifluorobiphenyl) - dibenzothiophen (F4-BPBTB), 2,6-di (4 - 3',5' - bifluorobiphenyl) - benzo[1,2-b:4,5-*b'*] bithiophene (F4-BPTBT), 2,5 - di (4 - 3',5' - bifluorobiphenyl) - thieno[3,2-b]thiophene (F4-BPTT), 2,5 - di (4 - 3",5" - bifluoro - 1,1':4',1"-terphenyl) - thieno[3,2-b]thiophene (F4-3PTT).

**Table 5**

| Inducing layer molecule | semiconductor molecule | epitaxial-relationship |
|---|---|---|
| | VOPc | incommensurate epitaxy |
| BPPh | | commensurate epitaxy |
| | TiOPc | incommensurate epitaxy |
| | | commensurate epitaxy |
| | VOTE | incommensurate epitaxy |
| BPBTB | | commensurate epitaxy |
| | PbPc | commensurate epitaxy |
| | | incommensurate epitaxy |
| | VOPc | commensurate epitaxy |
| BPTBT | | incommensurate epitaxy |
| | TiOPc | commensurate epitaxy |
| | | incommensurate epitaxy |
| | VOPc | commensurate epitaxy |
| | | incommensurate epitaxy |
| | TiOPc | commensurate epitaxy |
| | | incommensurate epitaxy |
| BPTT | | commensurate epitaxy |
| | PbPc | incommensurate epitaxy |
| | SnPc | incommensurate epitaxy |
| 3PTT | VOPc | commensurate epitaxy |
| | | incommensurate epitaxy |
| | TiOPc | commensurate epitaxy |
| | | incommensurate epitaxy |
| F2-BPPh | VOPc | commensurate epitaxy |
| | | incommensurate epitaxy |
| | PbPc | commensurate epitaxy |
| | | incommensurate epitaxy |
| | VOPc | commensurate epitaxy |
| F2-BPBTB | | incommensurate epitaxy |
| | TiOPc | commensurate epitaxy |
| | | incommensurate epitaxy |
| | TiOPc | commensurate epitaxy |
| | | incommensurate epitaxy |
| F2-BPTT | VOPc | commensurate epitaxy |
| | | incommensurate epitaxy |
| | PbPc | commensurate epitaxy |
| | | incommensurate epitaxy |
| F2-3PTT | VOPc | commensurate epitaxy |
| | | incommensurate epitaxy |
| | SnPc | commensurate epitaxy |
| | | incommensurate epitaxy |
| F4-BPPh | VOPc | commensurate epitaxy |
| | | incommensurate epitaxy |
| | TiOPc | commensurate epitaxy |
| | | incommensurate epitaxy |
| F4-BPBTB | VOPc | commensurate epitaxy |
| | | incommensurate epitaxy |
| | TiOPc | commensurate epitaxy |
| | | incommensurate epitaxy |
| F4-BPTBT | VOPc | commensurate epitaxy |
| | | incommensurate epitaxy |
| | PbPc | commensurate epitaxy |
| | | incommensurate epitaxy |
| F4-BPTT | VOPc | incommensurate epitaxy |
| | | commensurate epitaxy |
| | TiOPc | incommensurate epitaxy |
| | | commensurate epitaxy |
| F4-3PTT | VOPc | commensurate epitaxy |
| | | incommensurate epitaxy |
| | TiOPc | commensurate epitaxy |
| | | incommensurate epitaxy |

The present invention is not limited to the above-mentioned embodiments. In general, the inducing layer materials for the use of Weak Epitaxy Growth disclosed in the present invention can be used in other organic semiconductor components to prepare components in two-dimensional or three-dimensional integrated device. These integrated device can be used in integrated circuits, active matrix display, sensors and photovoltaic cells. The electronic components based on the present invention are easy to be manufactured in large scale.

## Claims

1. A non-planar phthalocyanine with inducing layer consisting of inducing layer material represented by the following general formula 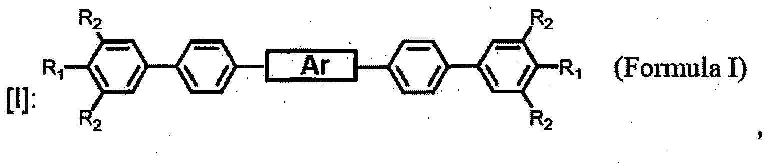 Wherein Ar is a conjugated aromatic group, selected from one of the following structures: 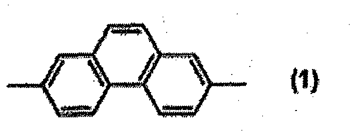 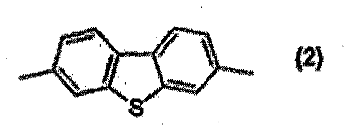 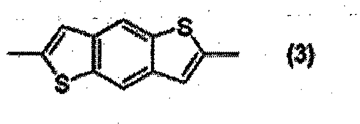 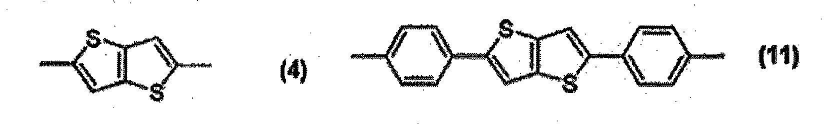 R₁ and R₂ in general formula I is hydrogen atom (H) or fluorine atom (F),according to the differences of the chemical structures of R₁ and R₂, the molecular structures of the new inducing layer materials conformed to general formula I can be divided into the following three types: (1) R₁= R₂=H , (2) R₁= F, R2=H, (3) R₁= H, R₂=F.

2. A non-planar metal phthalocyanine according to claim 1, **characterized in that**, the structures of the said inducing layer materials are as follows: any one of 2,7 - di (4 - biphenyl) -phenanthrene (BPPh), 2,7 - di (4 - biphenyl) - dibenzothiophen (BPBTB), 2,6 - di (4 - biphenyl) - benzo [1,2-*b* :4,5-*b'*] bithiophene(BPTBT), 2,5 - di (4 - biphenyl) - thieno[3,2-b]thiophene (BPTT), 2,5 - di (4 - 1,1':4',1" - terphenyl) - thieno[3,2-b]thiophene (3PTT), 2,7 - di (4 - 4'-fluorobiphenyl) - phenanthrene (F2-BPPh), 2,7 - di (4 - 4' - fluorobiphenyl)-dibenzothiophen (F2-BPBTB), 2,6 - di (4 - 4' - fluorobiphenyl) - benzo[1,2-b:4,5-*b'*] bithiophene (F2-BPTBT), 2,5 - di (4 - 4' - fluorobiphenyl) - thieno[3,2-b]thiophene(F2-BPTT), 2,5 - di (4 - 4" - fluoro - 1,1':4',1" - terphenyl)) - thieno[3,2-b]thiophene (F2-3PTT), 2,7 - di (4 - 3',5' - bifluorobiphenyl) - phenanthrene (F4-BPPh), 2,7 - di (4 - 3',5' - bifluorobiphenyl) - dibenzothiophen (F4-BPBTB), 2,6 - di (4 - 3',5'-bifluorobiphenyl) - benzo[1,2-b:4,5-*b'*] bithiophene (F4-BPTBT), 2,5 - di (4 - 3',5' - bifluorobiphenyl) - thieno[3,2-b]thiophene (F4-BPTT), 2,5 - di (4 - 3",5" - bifluoro-1,1':4',1" - terphenyl) - thieno[3,2-b]thiophene (F4-3PTT).

3. use of inducing layer materials in inducing weak epitaxial films of non-planar metal phthalocyanine, **characterized in that**, the inducing layer materials are represented by the following general formula: 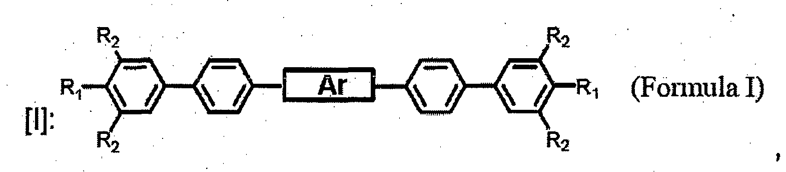 Wherein Ar is a conjugated aromatic group, selected from one of the following structures: 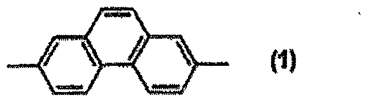 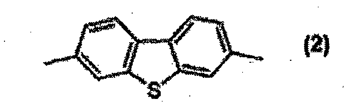 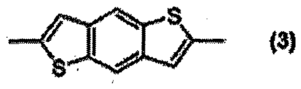 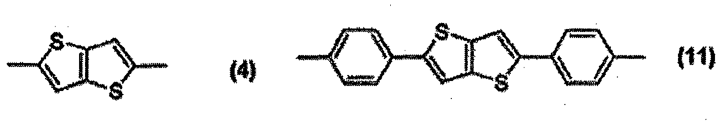 R₁ and R₂ in general formula I is hydrogen atom (H) or fluorine atom (F),according to the differences of the chemical structures of R₁ and R₂ , the molecular structures of the new inducing layer materials conformed to general formula I can be divided into the following three types: (1) R₁= R₂=H, (2) R₁= F, R₂=H, (3) R₁= H, R₂=F.

4. The use according to claim 3, **characterized in that**, the structures of the said inducing layer materials are as follows:
any one of 2,7 - di (4 - biphenyl) -phenanthrene (BPPh), 2,7 - di (4 - biphenyl) - dibenzothiophen (BPBTB), 2,6 - di (4 - biphenyl) - benzo [1,2-b :4,5-*b'*] bithiophene(BPTBT), 2,5 - di (4 - biphenyl) - thieno[3,2-b]thiophene (BPTT), 2,5 - di (4 - 1,1':4',1" - terphenyl) - thieno[3,2-b]thiophene (3PTT), 2,7 - di (4 - 4' - fluorobiphenyl) - phenanthrene (F2-BPPh), 2,7 - di (4 - 4' - fluorobiphenyl) - dibenzothiophen (F2-BPBTB), 2,6 - di (4 - 4' - fluorobiphenyl) - benzo[1,2-b:4,5-b'] bithiophene (F2-BPTBT), 2,5 - di (4 - 4' - fluorobiphenyl) - thieno[3,2-b]thiophene(F2-BPTT) 2,5 - di (4 - 4" - fluoro - 1,1':4',1" - terphenyl)) - thieno[3,2-b]thiophene (F2-3PTT), 2,7 - di (4 - 3',5' - bifluorobiphenyl) - phenanthrene (F4-BPPh), 2,7 - di (4 - 3',5' - bifluorobiphenyl) - dibenzothiophen (F4-BPBTB), 2,6 - di (4 - 3',5'-bifluorobiphenyl) - benzo[1,2-b:4,5-*b'*] bithiophene (F4-BPTBT), 2,5 - di (4 - 3',5'-bifluorobiphenyl) - thieno[3,2-b]thiophene (F4-BPTT), 2,5 - di (4 - 3",5" - bifluoro - 1,1':4',1" - terphenyl) - thieno[3,2-b]thiophene (F4-3PTT).

## Patentansprüche

1. Nicht-planares Phthalocyanin mit induzierender Schicht bestehend aus induzierendem Schichtmaterial, das durch die folgende allgemeine Formel dargestellt wird: 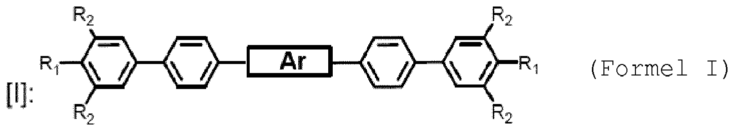 wobei Ar eine konjugierte aromatische Gruppe ist, die aus einer der folgenden Strukturen ausgewählt ist: 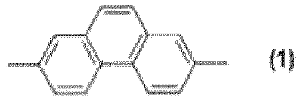 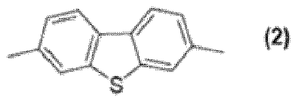 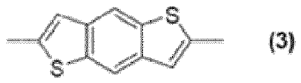 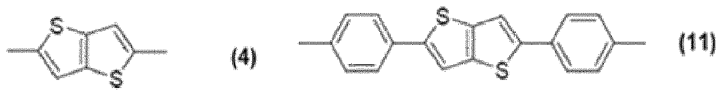 R₁ und R₂ in der allgemeinen Formel I je nach den Unterschieden der chemischen Strukturen von R₁ und R₂ Wasserstoffatom (H) oder Fluoratom (F) sind, wobei die molekularen Strukturen der neuen induzierenden Schichtmaterialien, die an die allgemeine Formel I angepasst sind, in die folgenden drei Typen unterteilt werden können: (1) R₁ = R₂ = H, (2) R₁ = F, R₂ = H, (3) R₁ = H, R₂ = F.

2. Nicht-planares Metallphthalocyanin nach Anspruch 1, **dadurch gekennzeichnet, dass** die Strukturen der induzierenden Schichtmaterialien sind, wie folgt:
ein beliebiges von 2,7-Di(4-biphenyl)-phenanthren (BPPh), 2,7-Di(4-biphenyl)dibenzothiophen (BPBTB), 2,6-Di(4-biphenyl)-benzo[1,2-b:4,5-b']bithiophen (BPTBT), 2,5-Di(4-biphenyl)-thieno[3,2-b]thiophen (BPTT), 2,5-Di(4-1,1':4',1"-terphenyl)-thieno[3,2-b]thiophen (3PTT), 2,7-Di(4-4'-fluorbiphenyl)-phenanthren (F2-BPPh), 2,7-Di(4-4'-fluorbiphenyl)dibenzothiophen (F2-BPBTB), 2,6-Di(4-4'-fluorbiphenyl)-benzo[1,2-b:4,5-*b*']bithiophen (F2-BPTBT), 2,5-Di(4-4'-fluorbiphenyl)-thieno[3,2-b]thiophen (F2-BPTT), 2,5-Di(4-4"-fluor-1,1':4',1"-terphenyl))-thieno[3,2-b]thiophen (F2-3PTT), 2,7-Di(4-3',5'- bifluorbiphenyl)-phenanthren (F4-BPPh), 2,7-Di(4-3',5'-bifluorbiphenyl)-dibenzothiophen (F4-BPBTB), 2,6-Di(4-3',5'-bifluorbiphenyl)-benzo[1,2-b:4,5-b']bithiophen (F4-BPTBT), 2,5-Di(4-3',5'-bifluorbiphenyl)-thieno[3,2-b]thiophen (F4-BPTT), 2,5-Di(4-3",5"-bifluor-1,1':4',1"-terphenyl)-thieno[3,2-b]thiophen (F4-3PTT).

3. Verwendung von induzierenden Schichtmaterialien beim Induzieren von schwachen Epitaxialfilmen aus nicht-planarem Metallphthalocyanin, **dadurch gekennzeichnet, dass** die induzierenden Schichtmaterialien durch die folgende allgemeine Formel dargestellt werden: 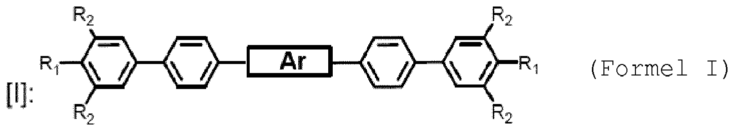 wobei Ar eine konjugierte aromatische Gruppe ist, die aus einer der folgenden Strukturen ausgewählt ist: 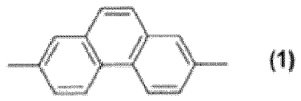 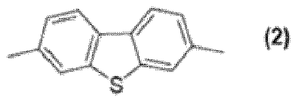 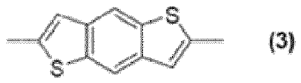 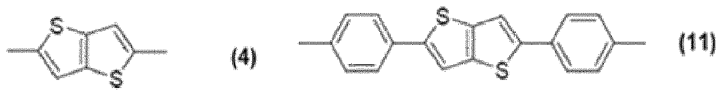 R₁ und R₂ in der allgemeinen Formel I je nach den Unterschieden der chemischen Strukturen von R₁ und R₂ Wasserstoffatom (H) oder Fluoratom (F) sind, wobei die molekularen Strukturen der neuen induzierenden Schichtmaterialien, die an die allgemeine Formel I angepasst sind, in die folgenden drei Typen unterteilt werden können: (1) R₁ = R₂ = H, (2) R₁ = F, R₂ = H, (3) R₁ = H, R₂ = F.

4. Verwendung nach Anspruch 3, **dadurch gekennzeichnet, dass** die Strukturen der induzierenden Schichtmaterialien sind, wie folgt:
ein beliebiges von 2,7-Di(4-biphenyl)-phenanthren (BPPh), 2,7-Di(4-biphenyl)dibenzothiophen (BPBTB), 2,6-Di(4-biphenyl)-benzo[1,2-b:4,5-b']bithiophen (BPTBT), 2,5-Di(4-biphenyl)-thieno[3,2-b]thiophen (BPTT), 2,5-Di(4-1,1':4',1"-terphenyl)-thieno[3,2-b]thiophen (3PTT), 2,7-Di(4-4'-fluorbiphenyl)-phenanthren (F2-BPPh), 2,7-Di(4-4'-fluorbiphenyl)dibenzothiophen (F2-BPBTB), 2,6-Di(4-4'-fluorbiphenyl)-benzo[1,2-b:4,5-*b*']bithiophen (F2-BPTBT), 2,5-Di(4-4'-fluorbiphenyl)-thieno[3,2-b]thiophen (F2-BPTT), 2,5-Di(4-4"-fluor-1,1':4',1"-terphenyl))-thieno[3,2-b]thiophen (F2-3PTT), 2,7-Di(4-3',5'- bifluorbiphenyl)-phenanthren (F4-BPPh), 2,7-Di(4-3',5'-bifluorbiphenyl)-dibenzothiophen (F4-BPBTB), 2,6-Di(4-3',5'-bifluorbiphenyl)-benzo[1,2-b:4,5-*b*']bithiophen (F4-BPTBT), 2,5-Di(4-3',5'-bifluorbiphenyl)-thieno[3,2-b]thiophen (F4-BPTT), 2,5-Di(4-3",5"-bifluor-1,1':4',1"-terphenyl)-thieno[3,2-b]thiophen (F4-3PTT).

## Revendications

1. Phtalocyanine non planaire avec couche d'induction se composant d'un matériau de couche d'induction représenté par la formule générale suivante 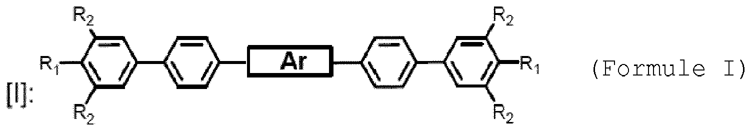 dans laquelle Ar est un groupe aromatique conjugué choisi parmi une des structures suivantes : 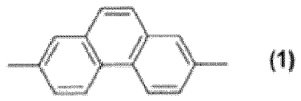 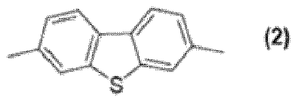 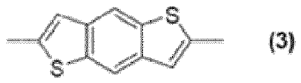 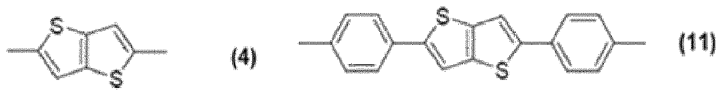 R₁ et R₂ dans la formule générale I est un atome d'hydrogène (H) ou un atome de fluor (F), en fonction des différences des structures chimiques de R₁ et R₂, les structures moléculaires des nouveaux matériaux de couche d'induction conformément à la formule générale I peuvent être divisés en les trois types suivants : (1) R₁ = R₂ = H, (2) R₁ = F, R₂ = H, (3) R₁ = H, R₂ = F.

2. Phtalocyanine métallique non planaire selon la revendication 1, **caractérisée en ce que** les structures desdits matériaux de couche d'induction sont les suivantes :
l'une quelconque de 2,7-di(4-biphényl)-phénanthrène (BPPh), 2,7-di(4-biphényl)-dibenzothiophène (BPBTB), 2,6-di(4-biphényl)-benzo[1,2-b:4,5-*b*']bithiophène (BPTBT), 2,5-di(4-biphényl)-thiéno[3,2-b]thiophène (BPTT), 2,5-di(4-1,1':4',1"-terphényl)-thiéno[3,2-b]thiophène (3PTT), 2,7-di(4-4'-fluorobiphényl)-phénanthrène (F2-BPPh), 2,7-di(4-4'-fluorobiphényl)-dibenzothiophène (F2-BPBTB), 2,6-di(4-4'-fluorobiphényl)-benzo[1,2-b:4,5-*b*']bithiophène (F2-BPTBT), 2,5-di(4-4'-fluorobiphényl)-thiéno[3,2-b]thiophène(F2-BPTT), 2,5-di(4-4"-fluoro-1,1':4',1"-terphényl))-thiéno[3,2-b]thiophène (F2-3PTT), 2,7-di(4-3',5'-bifluorobiphényl)-phénanthrène (F4-BPPh), 2,7-di(4-3',5'-bifluorobiphényl)-dibenzothiophène (F4-BPBTB), 2,6-di(4-3',5'-bifluorobiphényl)-benzo[1,2-b:4,5-*b*']bithiophène (F4-BPTBT), 2,5-di(4-3',5'-bifluorobiphényl)-thiéno[3,2-b]thiophène (F4-BPTT), 2,5-di(4-3",5"-bifluoro-1,1':4',1"-terphényl)-thiéno[3,2-b]thiophène (F4-3PTT).

3. Utilisation de matériaux de couche d'induction pour l'induction de films épitaxiaux minces de phtalocyanine métallique non planaire, **caractérisée en ce que**, les matériaux de couche d'induction sont représentés par la formule générale suivante : 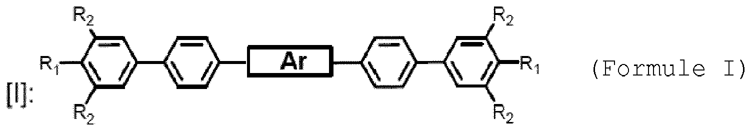 dans laquelle Ar est un groupe aromatique conjugué choisi parmi une des structures suivantes : 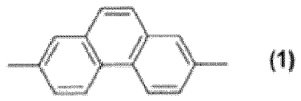 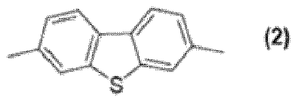 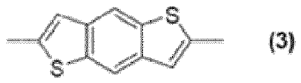 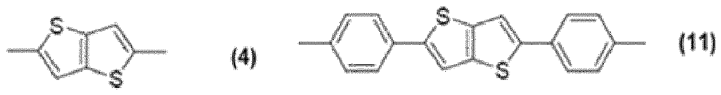 R₁ et R₂ dans la formule générale I est un atome d'hydrogène (H) ou un atome de fluor (F), en fonction des différences des structures chimiques de R₁ et R₂, les structures moléculaires des nouveaux matériaux de couche d'induction conformément à la formule générale I peuvent être divisés en les trois types suivants : (1) R₁ = R₂ = H, (2) R₁ = F, R₂ = H, (3) R₁ = H, R₂ = F.

4. Utilisation selon la revendication 3, **caractérisée en ce que** les structures desdits matériaux de couche d'induction sont les suivantes :
l'une quelconque de 2,7-di(4-biphényl)-phénanthrène (BPPh), 2,7-di(4-biphényl)-dibenzothiophène (BPBTB), 2,6-di(4-biphényl)-benzo[1,2-b:4,5-*b*']bithiophène (BPTBT), 2,5-di(4-biphényl)-thiéno[3,2-b]thiophène (BPTT), 2,5-di(4-1,1':4',1"-terphényl)-thiéno[3,2-b]thiophène (3PTT), 2,7-di(4-4'-fluorobiphényl)-phénanthrène (F2-BPPh), 2,7-di(4-4'-fluorobiphényl)-dibenzothiophène (F2-BPBTB), 2,6-di(4-4'-fluorobiphényl)-benzo[1,2-b:4,5-*b*']bithiophène (F2-BPTBT), 2,5-di(4-4'-fluorobiphényl)-thiéno[3,2-b]thiophène (F2-BPTT), 2,5-di(4-4"-fluoro-1,1':4',1"-terphényl))-thiéno[3,2-b]thiophène (F2-3PTT), 2,7-di(4-3',5'-bifluorobiphényl)-phénanthrène (F4-BPPh), 2,7-di(4-3',5'-bifluorobiphényl)-dibenzothiophène (F4-BPBTB), 2,6-di(4-3',5'-bifluorobiphényl)-benzo[1,2-b:4,5-*b*']bithiophène (F4-BPTBT), 2,5-di(4-3',5'-bifluorobiphényl)-thiéno[3,2-b]thiophène (F4-BPTT), 2,5-di(4-3",5"-bifluoro-1,1':4',1"-terphényl)-thiéno[3,2-b]thiophène (F4-3PTT).
